# EUROPÄISCHE PATENTSCHRIFT

(11) **EP 0 723 952 B1**
(45) Veröffentlichungstag und Bekanntmachung des Hinweises auf die Patenterteilung: **20.09.2000**
(21) Anmeldenummer: 96100822.4
(22) Anmeldetag: 20.01.1996
(51) Int. Cl.: C07C 209/84, C07C 211/08

(54) **Verfahren zur Reinigung von tertiären Fettalkylmethylaminen**
Process for the purification of tertiary fatty alkyl methyl amines
Procédé pour la purification d'amines grasses alkylées méthyliques tertiaires

(30) Priorität: 27.01.1995 DE 19502545; 07.03.1995 DE 19507987
(43) Veröffentlichungstag der Anmeldung: 31.07.1996
(73) Patentinhaber: Clariant GmbH, 65929 Frankfurt am Main (DE)
(72) Erfinder: Papenfuhs, Bernd, Dr., D-84524 Neuötting (DE); Seitz, Hubert, Dr., D-84508 Burgkirchen (DE); Gallas, Andreas, DI (FH), D-84567 Erlbach (DE)

(56) Entgegenhaltungen:
- BE-A- 859 665
- US-A- 2 461 191
- US-A- 3 441 558
- US-A- 3 472 740
- US-A- 4 138 437
- US-A- 4 845 289

## Beschreibung

Die Erfindung betrifft ein Verfahren zur Reinigung von tertiären Fettalkylmethylaminen, insbesondere von Fettalkyldimethylaminen, die durch Alkylierung von Mono- und/oder Dimethylamin erhalten worden sind.

Tertiäre Fettalkylmethylamine wie Difettalkylmonomethylamin oder Monofettalkyldimethylamin (Fettalkyldimethylamin) werden bekanntlich vorteilhaft durch Alkylierung von Mono- und/oder Dimethylamin mit Fettalkoholen oder Fettalkylhalogeniden hergestellt. Zur Aufarbeitung des Reaktionsproduktes wird der eingesetzte Katalysator abfiltriert und das tertiäre Fettalkylmethylamin von hochsiedenden Nebenprodukten durch Destillation abgetrennt und gewonnen.

Solche Alkylierungsverfahren von Mono- und/oder Dimethylamin sind zum Beispiel in US-A-4 138 437 und in GB-A-1 585 480 beschrieben. Was die Reinheit der gewonnenen tertiären Fettalkylmethylamine betrifft, so enthalten sie im allgemeinen neben nicht umgesetztem Alkylierungsmittel auch primäre und sekundäre Amine in einer Menge von insgesamt etwa < 1,5 Gew.-%, bezogen auf tertiäres Fettalkylmethylamin (vergleiche zum Beispiel US-A-4 138 437, Beispiel 1).

In US-A-2 461 191 wird die destillative Trennung von Mischungen aus Di- und Trimethylamin beschrieben.

Aus US-A-4 845 289 geht hervor, daß es schwierig sei, Trimethylamin aus wäßrigen Lösungen mittels Durchleiten von Luft abzutrennen.

Es hat sich nun herausgestellt, daß tertiäre Fettalkylmethylamine der beschriebenen Art gelegentlich einen mehr oder weniger starken Fischgeruch abgeben, und zwar nahezu unabhängig vom Gehalt an Mono- und Dimethylamin.

Durch sorgfältige Untersuchungen von riechenden Fettalkyldimethylaminen wurde gefunden, daß die den Fischgeruch verursachende Substanz im wesentlichen Trimethylamin sein dürfte. Die Geruchsschwelle von Trimethylamin ist etwa 100 bis 200mal niedriger als jene von Mono- und Dimethylamin und die Siedepunkte der drei Amine bei Atmosphärendruck liegen wie folgt: Monomethylamin -6,32 °C, Dimethylamin +6,88 °C und Trimethylamin +2,87 °C.

Es wurde nun ein Verfahren gefunden, mit dem es in einfacher und wirtschaftlicher Weise möglich ist, Trimethylamin von tertiären Fettalkylmethylaminen abzutrennen und gleichzeitig eine Geruchsverbesserung zu erhalten.

Das erfindungsgemäße Verfahren ist dadurch gekennzeichnet, daß man das zu reinigende tertiäre C₆-C₂₄-Fettalkylmethylamin in flüssiger Phase einem Vakuum oder einem inerten Gasstrom aussetzt.

Die Analyse von erfindungsgemäß behandelten Fettalkyldimethylaminen zeigt unerwarteterweise, daß sie nach wie vor im wesentlichen die gleiche Menge an Mono- und Dimethylamin enthalten. Dies ist im Hinblick auf die Siedepunkte von Monomethylamin (-6,32 °C), Dimethylamin (6,88 °C) und Trimethylamin (2,87 °C) in der Tat ein überraschendes Ergebnis. Es war demnach nicht zu erwarten, daß mit dem erfindungsgemäßen Verfahren gerade der Trimethylamingehalt reduziert werden kann und ein diesbezüglich gereinigtes Fettalkyldimethylamin erhalten wird.

Beim erfindungsgemäßen Verfahren soll das zu behandelnde tertiäre Fettalkylmethylamin im wesentlichen flüssig vorliegen. Ist es zum Beispiel bei Raumtemperatur und Atmosphärendruck von sich aus flüssig, ist eine weitere Erwärmung nicht erforderlich. Um flüssigen Zustand und eine möglichst kurze Behandlungsdauer zu gewährleisten, wird man das zu reinigende tertiäre Fettalkylmethylamin im allgemeinen bei einer Temperatur von 10 bis 200 °C halten, vorzugsweise von 20 bis 170 °C. Auch bei den genannten höheren Temperaturen, die besonders kurze Behandlungszeiten ermöglichen, treten Zersetzungen nicht auf. Das flüssige Produkt wird erfindungsgemäß einem Vakuum unterworfen, gegebenenfalls unter Rühren, bis der angestrebte Effekt erreicht ist. Die Höhe des Vakuums kann innerhalb weiter Grenzen variieren und hängt im wesentlichen von der Zeit ab, in der man den Reinigungseffekt erreichen möchte. So erfordern kurze Behandlungszeiten ein höheres Vakuum und umgekehrt. Ein vorteilhaftes Vakuum, das an flüssiges Produkt gegebenenfalls unter Rühren angesetzt und aufrechterhalten wird, liegt bei 5·10² bis 5·10⁴ Pa (5 bis 500 mbar), vorzugsweise bei 10³ bis 3·10⁴ Pa (10 bis 300 mbar).

Der angestrebte Reinigungseffekt wird erfindungsgemäß auch dadurch erreicht, daß man das flüssige tertiäre Fettalkylmethylamin gegebenenfalls unter Rühren mit einem Inertgas wie Stickstoff, Argon und/oder Helium behandelt. Als Inertgasstrom kann gegebenenfalls auch Luft eingesetzt werden. Die Menge an Inertgas kann innerhalb weiter Grenzen variieren und hängt ebenfalls im wesentlichen von der Zeit ab, in der man die gewünschte Trimethylamin-Reduzierung erreichen möchte, wobei auch hier kurze Behandlungszeiten einen an Menge und Geschwindigkeit höheren Gasstrom erfordern und umgekehrt. Ein vorteilhafter Inertgasstrom liegt bei 1 bis 300 l, vorzugsweise bei 5 bis 200 l, Inertgas pro Stunde und pro Kilogramm zu behandelndes tertiäres Fettalkylmethylamin. Es ist bevorzugt, das Inertgas gegebenenfalls unter Rühren durch das flüssige Produkt hindurchzuführen, was zum Beispiel durch ein in das Produkt mehr oder weniger tief eingetauchtes Gasrohr erreicht werden kann. Die erfindungsgemäße Behandlung mit einem Inertgasstrom zur Entfernung von Trimethylamin wird bevorzugt bei Atmosphärendruck oder unter Anwendung eines Vakuums durchgeführt, wobei bezüglich der Höhe des Vakuums auch hier das oben Gesagte gilt. Die Inertgasstrombehandlung kann auch bei Vorliegen eines Systemüberdruckes durchgeführt werden, wobei der Inertgasstrom den Systemdruck zu überwinden hat.

Die Kombination des erfindungsgemäßen Verfahrens mit anderen Reinigungsschritten, wie zum Beispiel einer Destillation, ist möglich und nicht an eine bestimmte Reihenfolge gebunden. Das erfindungsgemäße Verfahren stellt eine einfache und wirtschaftliche Methode zur Reinigung und Geruchsverbesserung von tertiärem Fettalkylmethylamin dar, unabhängig von der Herstellungsart des Fettalkylmethylamins. Es können sowohl größere Mengen als auch nur Spuren von Trimethylamin bis auf unter die Nachweisgrenze entfernt werden. Das erfindungsgemäß behandelte Produkt enthält im allgemeinen nur noch < 1 ppm Trimethylamin. Die Behandlungszeit liegt bei allen Varianten des erfindungsgemäßen Verfahrens im allgemeinen im Bereich von 30 Minuten bis 10 Stunden. Ein weiterer Vorteil des erfindungsgemäßen Verfahrens liegt darin, daß auch die Folgeprodukte der gereinigten tertiären Fettalkylmethylamine geruchsverbessert anfallen. Folgeprodukte sind zum Beispiel Aminoxide und Betaine sowie daraus hergestellte Formulierungen, beispielsweise für den Kosmetiksektor, Reinigungssektor, Waschmittelsektor und dergleichen.

Die im Rahmen der vorliegenden Erfindung zu behandelnden tertiären Fettalkylmethylamine sind in den eingangs genannten Druckschriften ausführlich beschrieben. Es handelt sich um solche der nachstehenden Formel 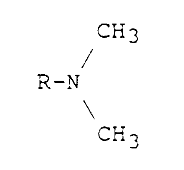 worin R einen Alkylrest oder einen Alkenylrest mit 6 bis 24 C-Atomen, vorzugsweise 8 bis 18 C-Atomen, bedeutet. Der Alkenylrest enthält im allgemeinen 1 bis 3 Doppelbindungen. R stellt häufig auch eine Mischung von Alkyl- und/oder Alkenylresten dar, zum Beispiel C₁₂ und C₁₄-Alkyl (C_{12/14}-Alkyl), C₁₂ und C₁₄-Alkenyl (C_{12/14}-Alkenyl) oder von C₁₂ bis C₁₈-Alkyl und/oder -Alkenyl. Beispiele für Alkyl- und Alkenylgruppen sind Octyl, Octenyl, Decyl, Decenyl, Dodecyl (Lauryl), Dodecenyl, Stearyl, Oleyl, Cocosalkyl und Talgfettalkyl.

Die Erfindung wird nun an Beispielen noch näher erläutert.

### Beispiel 1

Im Beispiel 1 von US-A-4 138 437 wird Dimethylamin mit Dodecanol-1 in Gegenwart von Wasserstoff und Kupferchromit als Katalysator umgesetzt. Zur Aufarbeitung des Reaktionsproduktes wird vom Katalysator abfiltriert und ein Dodecyldimethylamin als Rohprodukt gewonnen. Das Rohprodukt wird zur Abtrennung von hochsiedenden Nebenprodukten einer Vakuumdestillation unterworfen. Das nach Abnahme eines Vorlaufs erhaltene destillierte C₁₂-Alkyldimethylamin enthält etwa 140 ppm Monomethylamin, 150 ppm Dimethylamin und etwa 64 ppm Trimethylamin und hat einen mehr oder weniger unangenehmen Geruch.

200 g von dem riechenden (destillierten) C₁₂-Alkyldimethylamin werden in einem mit Rührer und Thermometer ausgestatteten Reaktionsgefäß vorgelegt, auf 40 °C erwärmt und bei dieser Temperatur und einem Vakuum von 2,5·10⁴ Pa (250 mbar) langsam gerührt. 2 Stunden nach Beginn dieser Behandlung ist das C₁₂-Alkyldimethylamin deutlich geruchsverbessert. Es ist kein Trimethylamin (TMA) mehr nachweisbar (Nachweisgrenze: 1 ppm); Monomethylamin (MMA) und Dimethylamin (DMA) sind dagegen nicht ausgetragen worden, ihr Gehalt ist vielmehr nahezu konstant geblieben, wie die nachstehende Tabelle 1 zeigt:

**Tabelle 1**

| Zeit [Minuten] | MMA [ppm] | DMA [ppm] | TMA [ppm] |
|---|---|---|---|
| 0 | 140 | 150 | 64 |
| 30 | 140 | 150 | 12 |
| 60 | 139 | 148 | 3 |
| 120 | 138 | 147 | < 1 |

### Beispiel 2

Das im Beispiel 1 erwähnte Rohprodukt enthält etwa 150 ppm Monomethylamin und etwa 160 ppm Dimethylamin, aber 1300 ppm Trimethylamin. Es hat einen im Vergleich zum destillierten Dodecyldimethylamin viel stärker hervortretenden unangenehmen Geruch. Es wird der erfindungsgemäßen Reinigung unterworfen.

200 g von dem stark riechenden Rohprodukt C₁₂-Alkyldimethylamin werden in einem mit Rührer und Thermometer ausgestatteten Reaktionsgefäß vorgelegt, auf 50 °C erwärmt und bei dieser Temperatur und einem Vakuum von 5·10³ Pa (50 mbar) langsam gerührt. Nach 3 Stunden wird ein geruchsverbessertes C₁₂-Alkyldimethylamin erhalten. Der ursprüngliche Gehalt von 1300 ppm Trimethylamin (TMA) ist auf unter die Nachweisegrenze reduziert worden; der Gehalt an Monomethylamin (MMA) und Dimethylamin (DMA) ist dagegen nahezu unverändert geblieben, wie die nachstehende Tabelle 2 zeigt:

**Tabelle 2**

| Zeit [Minuten] | MMA [ppm] | DMA [ppm] | TMA [ppm] |
|---|---|---|---|
| 0 | 150 | 160 | 1300 |
| 120 | 145 | 158 | 5 |
| 180 | 143 | 155 | < 1 |

### Beispiel 3

200 g von einem riechenden C_{8/10}-Alkyldimethylamin werden in einem mit Rührer, Thermometer und Gaseinleitungsrohr ausgestatteten Reaktionsgefäß vorgelegt und 2 Stunden lang bei 40 °C unter Durchleiten eines Stickstoffstromes von 30 l pro Stunde langsam gerührt (das Stickstoffeinleitungsrohr ist durch das vorgelegte Alkyldimethylamin hindurch bis zum Boden des Reaktionsgefäßes positioniert). Nach den 2 Stunden erfindungsgemäßer Behandlung hat das C_{8/10}-Alkyldimethylamin die in der nachstehenden Tabelle 3 zusammengefaßten Endwerte an Monomethylamin (MMA), Dimethylamin (DMA) und Trimethylamin (TMA); in der Tabelle 3 sind auch die Ausgangswerte angegeben:

**Tabelle 3**

| Zeit [Minuten] | MMA [ppm] | DMA [ppm] | TMA [ppm] |
|---|---|---|---|
| 0 | 70 | 230 | 164 |
| 120 | 69 | 225 | < 1 |

### Beispiel 4

Beispiel 3 wird wiederholt, wobei 200 g von einem riechenden C_{16/18}-Alkyldimethylamin eingesetzt werden. In der nachstehenden Tabelle 4 sind die Anfangs- und Endwerte des gereinigten Aminproduktes zusammengefaßt:

**Tabelle 4**

| Zeit [Minuten] | MMA [ppm] | DMA [ppm] | TMA [ppm] |
|---|---|---|---|
| 0 | 69 | 148 | 14 |
| 120 | 64 | 136 | < 1 |

### Beispiel 5

Beispiel 1 wird wiederholt mit dem Unterschied, daß anstelle von 40 °C auf 120 °C erwärmt und bei dieser Temperatur und einem Vakuum von 2,5·10⁴ Pa (250 mbar) langsam gerührt wird. Schon nach 1 Stunde nach Beginn dieser Behandlung ist das C₁₂-Alkyldimethylamin deutlich geruchsverbessert. Es ist kein Trimethylamin (TMA) mehr nachweisbar (Nachweisgrenze: 1 ppm); Monomethylamin (MMA) und Dimethylamin (DMA) sind dagegen nicht ausgetragen worden, ihr Gehalt ist vielmehr nahezu konstant geblieben, wie die nachstehende Tabelle 5 zeigt:

**Tabelle 5**

| Zeit [Minuten] | MMA [ppm] | DMA [ppm] | TMA [ppm] |
|---|---|---|---|
| 0 | 140 | 150 | 64 |
| 60 | 137 | 145 | < 1 |

### Beispiel 6

Beispiel 3 wird wiederholt mit dem Unterschied, daß 1 Stunde lang (anstelle von 2 Stunden) bei 80 °C (anstelle von 40 °C) unter Durchleiten eines Stickstoffstromes von 30 l pro Stunde langsam gerührt wird. Schon nach 1 Stunde Behandlung hat das C_{8/10}-Alkyldimethylamin die in der nachstehenden Tabelle 6 zusammengefaßten Endwerte an Monomethylamin (MMA), Dimethylamin (DMA) und Trimethylamin (TMA); in der Tabelle 6 sind auch die Ausgangswerte angegeben:

**Tabelle 6**

| Zeit [Minuten] | MMA [ppm] | DMA [ppm] | TMA [ppm] |
|---|---|---|---|
| 0 | 70 | 230 | 164 |
| 60 | 67 | 220 | < 1 |

### Beispiel 7

Es wird das im Beispiel 4 genannte riechende C_{16/18}-Alkyldimethylamin eingesetzt und nach Beispiel 3 behandelt mit dem Unterschied, daß 1 Stunde lang (anstelle von 2 Stunden) bei 180 °C (anstelle von 40 °C) unter Durchleiten eines Stickstoffstromes von 30 l pro Stunde gerührt wird. In der nachstehenden Tabelle 7 sind die Anfangs- und Endwerte des gereinigten Aminproduktes zusammengefaßt:

**Tabelle 7**

| Zeit [Minuten] | MMA [ppm] | DMA [ppm] | TMA [ppm] |
|---|---|---|---|
| 0 | 69 | 148 | 14 |
| 50 | 60 | 132 | < 1 |

## Patentansprüche

1. Verfahren zur Reinigung von tertiären C₆-C₂₄-Fettalkyldimethylaminen, dadurch gekennzeichnet, daß man das zu reinigende tertiäre Fettalkyldimethylamin in flüssiger Phase ohne Destillation einem Vakuum oder einem inerten Gasstrom aussetzt.

2. Verfahren nach Anspruch 1, dadurch gekennzeichnet, daß man das zu reinigende tertiäre Fettalkyldimethylamin einem Vakuum von 5·10² bis 5·10⁴ Pa (5 bis 500 mbar) aussetzt.

3. Verfahren nach Anspruch 1 oder 2, dadurch gekennzeichnet, daß man das zu reinigende tertiäre Fettalkyldimethylamin einem inerten Gasstrom bei einem Vakuum von 5·10² bis 5·10⁴ Pa (5 bis 500 mbar) aussetzt.

4. Verfahren nach Anspruch 1, dadurch gekennzeichnet, daß das Fettalkyldimethylamin durch Alkylierung von Dimethylamin mit einem C₆-C₂₄ Fettalkohol hergestellt worden ist.

## Claims

1. A process for purifying tertiary C₆-C₂₄ fatty alkyldimethylamines, which comprises exposing, in the liquid phase without distillation, the tertiary fatty alkyldimethylamine to be purified to a vacuum or an inert gas stream.

2. The process as claimed in claim 1, wherein the tertiary fatty alkyldimethylamine to be purified is exposed to a vacuum of 5·10² to 5·10⁴ Pa (5 to 500 mbar).

3. The process as claimed in claim 1 or 2, wherein the tertiary fatty alkyldimethylamine to be purified is exposed to an inert gas stream at a vacuum of 5.10² to 5.10⁴ Pa (5 to 500 mbar).

4. The process as claimed in claim 1, wherein the fatty alkyldimethylamine was prepared by alkylation of dimethylamine with a C₆-C₂₄ fatty alcohol.

## Revendications

1. Procédé de purification d'(alkyl gras en C6-C₂₄)diméthylamines tertiaires, caractérisé en ce que l'on expose l'(alkyl gras)diméthylamine tertiaire à purifier en phase liquide, sans distillation, à un vide ou à un courant de gaz inerte.

2. Procédé selon la revendication 1, caractérisé en ce qu'on expose l'(alkyl gras)diméthylamine tertiaire à purifier à un vide de 5.10² à 5.10⁴ Pa (5 à 500 mbar).

3. Procédé selon la revendication 1 ou 2, caractérisé en ce qu'on expose l'(alkyl gras)diméthylamine tertiaire à purifier à un courant de gaz inerte sous un vide de 5.10² à 5.10⁴ Pa (5 à 500 mbar).

4. Procédé selon la revendication 1, caractérisé en ce que l'(alkyl gras)diméthylamine a été préparée par alkylation de la diméthylamine au moyen d'un alcool gras en C₆-C₂₄.
